(19) Europäisches Patentamt
European Patent Office
Office européen des brevets

(11) Publication number: 0 153 168 B1

(12) **EUROPEAN PATENT SPECIFICATION**

(45) Date of publication of patent specification: 20.09.89

(51) Int. Cl.$^4$: C 01 B 21/16

(21) Application number: 85301021.3

(22) Date of filing: 15.02.85

# (54) Process for preparing a hydrazine hydrohalide.

(30) Priority: 17.02.84 JP 28322/84
17.02.84 JP 28323/84

(43) Date of publication of application:
28.08.85 Bulletin 85/35

(45) Publication of the grant of the patent:
20.09.89 Bulletin 89/38

(84) Designated Contracting States:
DE FR GB

(56) References cited:
DE-A-2 848 002
US-A-2 870 206

CHEMICAL ABSTRACTS, vol. 85, no. 24, 13th December 1976, page 123, first column, abstract no. 179707r, Columbus, Ohio, US; HAYASHI et al.: "Ammonia-hydrazine conversion processes. 10. Hydrazine production from ammonia via azine"; IND .ENG .CHEM .PRO .RES .Dev., 1976, 15(4), 299-303

(73) Proprietor: MITSUBISHI GAS CHEMICAL COMPANY, INC.
5-2, Marunouchi 2-chome Chiyoda-Ku
Tokyo, 100 (JP)

(72) Inventor: Nawata, Takanari
No. 5-19-3 Kanamachi
Katsushika-ku Tokyo (JP)
Inventor: Kohzaki, Toshiaki
No. 2689-20 Kashiwada Ushikumachi
Inashiki-gun Ibaraki (JP)
Inventor: Sakaguchi, Shuzabu
No. 19-5 Toride-nishi 1-chome
Ibaraki (JP)
Inventor: Aoki, Osamu
No. 69 Takayanagishinden
Matsudo-shi Chiba (JP)
Inventor: Takeda, Norio
No. 277-28 Oaza Obusuma Showamachi
Kita Katsushika-gun Saitama (JP)
Inventor: Shimpo, Masafumi
No. 5-11-16 Kanamachi
Katsushika-ku Tokyo (JP)
Inventor: Aoki, Yoshiyuki
No. 635-298 Oaza Ushiku Ushikumachi
Inashiki-gun Ibaraki (JP)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 153 168 B1

Courier Press, Leamington Spa, England.

(56) References cited:
CHEMICAL ABSTRACTS, vol. 93, no. 15, 13th October 1980, page 693, column 2, abstract no. 149996g, Columbus, Ohio, US; TOMIYA et al.: "Benzophenone resins"; & JPN. KOKAI TOKKYO KOHO, 80 38,346, 17-03-1980; & JP - A - 38346/80 (Cat. D)

(74) Representative: Diamond, Bryan Clive et al
Gee & Co. Chancery House Chancery Lane
London WC2A 1QU (GB)

**Description**

This invention relates to a process for preparing hydrazine hydrohalides comprising oxidizing benzophenone-imines with molecular oxygen in the presence of a copper halide catalyst and hydrolyzing the resulting benzophenone-azines.

Hydrazine hydrohalides are industrially useful for a wide variety of applications, for example, as foaming agents or intermediates for producing hydrazine, and the like, and, therefore, it is desirable to be able to prepare them inexpensively.

It is known that benzophenone-imines are contacted with molecular oxygen in the presence of a copper halide catalyst to obtain benzophenone-azines as described in U.S. Patent 2,870,206 and that azines are hydrolyzed with an aqueous solution of a hydrohalic acid to obtain hydrazine hydrohalides as described in Japanese Patent Application (OPI) No. 97600/76 (the term "OPI" as used herein means an unexamined published patent application).

Production of hydrazine hydrohalides by combining these processes is industrially advantageous but inevitably demands a simple and easy process for the removal and reuse of the copper catalyst used.

Known processes for recovering the copper halide catalyst comprise adding water, aqueous ammonia or an aqueous salt solution to the reaction mixture containing the benzophenone-azines produced by oxidation with molecular oxygen to thereby precipitating the copper halide catalyst, and reusing the thus recovered catalyst as disclosed in Japanese Patent Applications (OPI) Nos. 71045/78 and 38346/80. These processes, however, cannot be considered industrially advantageous because of poor workability resulting from handling of a solid.

The present inventors have conducted extensive investigations on processes for preparing hydrazine hydrohalides by hydrolysis of a benzophenone-azine obtained by oxidation of a benzophenone-imine with molecular oxygen. As a result, it has now been found that, when benzophenone-azines are hydrolyzed by a specific process, the catalyst can not only easily be extracted and removed from the reaction mixture simultaneously with the hydrolysis but also simply and effectively be recovered from the resulting aqueous solution of hydrazine hydrohalide containing the catalyst and reused. The present invention is based on this finding.

The present invention relates to a process for preparing a hydrazine hydrohalide comprising the steps of:

(a) oxidizing a benzophenone-imine with molecular oxygen in the presence of a copper halide catalyst to prepare a benzophenone-azine;

(b) contacting the oxidation reaction mixture obtained in step (a) with an aqueous solution containing a hydrohalogenic acid at a concentration of from 10 to 60% by weight, thereby hydrolyzing the benzophenone-azine to produce a hydrazine hydrohalide and, at the same time, extracting and removing the catalyst with the hydrohalic acid-containing aqueous solution from the reaction mixture, to obtain an aqueous solution containing the hydrazine hydrohalide and the catalyst;

(c) separating the aqueous solution containing the hydrazine hydrohalide and catalyst which is obtained in step (b) from the hydrolysis mixture and adjusting the solution to a pH of from 3 to 7 with an alkali;

(d) contacting the aqueous solution having a pH of 3 to 7 obtained in step (c) with benzophenone-imine to extract the catalyst with the benzophenone-imine from said aqueous solution;

(e) separating the catalyst-containing benzophenone-imine solution obtained in step (d) from the aqueous solution containing the hydrazine hydrohalogenide, thereby to recover the hydrazine hydrohalogenide as an aqueous solution and to recycle the catalyst-containing benzophenone-imines solution to step (a).

According to the present invention, hydrazine hydrohalogenides can be produced with industrial advantages in terms of easiness to recover and reuse the catalyst.

In the accompanying drawing:

The figure is a flow sheet of the reaction used in Example 2. The elements are: supply pipe for benzophenone-imine 1, pipes for discharging air 2 and 8, first reactor 3, pipes for introducing air 4 and 10, pipes for discharging reaction solution 5 and 11, pump 6, pipe for introducing reaction solution 7, and second reactor 9.

The benzophenone-imines which can be used in the present invention can be represented by the formula (I):

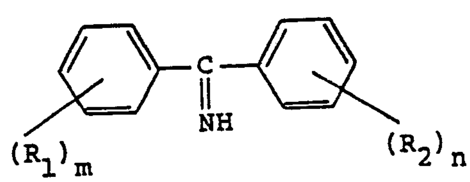

(I)

wherein $R_1$ and $R_2$, which may be the same or different, each represents an acyclic, alicyclic or aromatic hydrocarbon group having from 1 to 10 carbon atoms, an ether, acyl, acyloxy, alkoxycarbonyl, carboxylic

acid amido or di-substituted amino group derived from these hydrocarbon groups, a halogen atom, a hydroxyl group, a nitro group or a cyano group, or $R_1$ and $R_2$ may together form a single bond or a ring; and *m* and *n* each represents 0 or an integer of from 1 to 5.

Specific examples of the benzophenone-imines represented by the formula (I) include benzophenone-imine, 2-, 3- or 4-methylbenzophenone-imine, 2-, 3- or 4-ethylbenzophenone-imine, 2-, 3- or 4-n- and/or isopropylbenzophenoneimine, 2-, 3- or 4-n- and/or iso- and/or tert-butylbenzophenone-imine, 2-, 3- or 4-amylbenzophenone-imine, 2-, 3- or 4-decylbenzophenone-imine, 2-, 3- or 4-methoxybenzophenone-imine, 4-cyclohexylbenzophenone-imine, 4-phenylbenzophenone-imine, 2,4-dimethylbenzophenone-imine, 2,3-dimethylbenzophenone-imine, 3,4-dimethylbenzophenone-imine, 2,4-diethylbenzophenone-imine, 2,3-diethylbenzophenone-imine, 3,4-diethylbenzophenone-imine, 2-methyl-4-ethylbenzophenone-imine, 2-methyl-4-butylbenzophenone-imine, 2,2'-, 3,3'-, 4,4'-, 2,3'-, 2,4'- or 3,4'-dimethylbenzophenone-imine, 2-, 3- or 4-chlorobenzophenone-imine, 2-chloro-4-methylbenzophenone-imine, 4-chloro-4'-methylbenzophenone-imine, 4,4'-dichlorobenzophenone-imine, 4-nitrobenzophenone-imine, 2,4-dinitrobenzophenone-imine, 4-hydroxybenzophenone-imine, 4-N,N-dimethylaminobenzophenone-imine, 4-acetylbenzophenone-imine, 4-methoxybenzophenone-imine, 4-N,N-dimethylcarbamoylbenzophenone-imine, 4-cyanobenzophenone-imine, fluorenone-imine, xanthone-imine, anthrone-imine and acridone-imine.

Methods for preparing these and other imines include, for example, a method of reacting corresponding benzophenones with ammonia, a method of reacting benzonitriles with an aryl magnesium bromide, i.e., a Grignard reagent, a method of dehydrating a diarylamino alcohol, and the like. All imines prepared by any of these methods can be employed in the present invention.

The benzophenone-imines that can be used in the present invention, except for unsubstituted benzophenone-imine of the formula (I) wherein m and n are 0, are those containing various substituents or with substituents jointly forming a single bond or a ring. In carrying out the process of the invention on an industrial scale, unsubstituted benzophenone-imine and 1- or 2-mono- or 1,2-di-substituted benzophenone-imine are preferred. Among them, unsubstituted benzophenone-imine is particularly preferred.

The copper halide which is used as a catalyst in the present invention preferably is cuprous chloride, cuprous bromide or cuprous iodide, with cuprous chloride being more preferred.

Solvents are not particularly required in the present invention. It is possible, however, to use solvents for the purpose of maintaining the reaction system in a solution state or facilitating oil-water separation in the extraction, hydrolysis or back extraction. Such being the case, solvents that are not easily oxidized in the oxidation of benzophenone-imines and, in particular, have poor affinity for water and a low viscosity are preferred. Examples of such solvents are benzene, toluene, o-, m- or p-xylene, ethylbenzene, mesitylene, cumene, pseudocumene, amylbenzene, aromatic hydrocarbons having from 6 to 16 carbon atoms, and mixtures thereof, chlorobenzene, o-, m- or p-dichlorobenzene, nitrobenzene, o-, m- or p-dinitrobenzene, o-, m- or p-chlorotoluene, diphenyl, phenanthrene, anisole, diphenyl ether, acetophenone, dibenzyl, benzophenone, hexane, heptane, cyclohexane, cyclooctane, ethylcyclohexane, ethylene dichloride, tetrachloroethylene, diisopropyl ether, dipropyl ether, diisobutyl ketone, butyl acetate, butyl benzoate, phenyl benzoate and dimethyl phthalate. Of these, benzophenone is most preferred. It is usually preferable in the present invention to use an imination reaction mixture of benzophenones as the starting benzophenone-imines, and reaction mixtures having benzophenone-imine concentrations of from 1 to 90% by weight, and preferably from 5 to 50% by weight, are usually employed. In case of using the imination reaction mixture, solvent is not always necessary since the unreacted benzophenones remaining in the reaction mixture serves as a solvent.

Each step of the process of the present invention will now be described in detail.

Step (a):

Conditions for the oxidation of benzophenone-imine are not definitely specified as they vary depending on the activity and amount of the catalyst added to the reaction system, and the like, but usually, the copper halide catalyst is used in an amount ranging from $10^{-2}$ to $2 \times 10^{-1}$ mols, and preferably from $2 \times 10^{-2}$ to $10^{-1}$ mols, per liter of the total volume of the reaction solution. The reaction temperature usually is from 60° to 250°C, and preferably from 70° to 230°C. The molecular oxygen which can be used includes pure oxygen, air, and other oxygen-containing mixed gases, and the reaction can be carried out either under atmospheric pressure or under elevated pressure. The oxygen partial pressure is preferably from 0.01 to 20 atm., and more preferably from 0.05 to 10 atm., but wider ranges may also be used. In order to minimize side reactions or sedimentation of the catalyst, it is desirable to control the oxidation reaction so that the conversion of the benzophenone-imine falls within the range of from 85% to 99%.

In this step (a), the reaction can preferably be carried out according to an embodiment hereinafter described in order to prevent sedimentation of the catalyst and to stably conduct the reaction.

Such a preferred embodiment of step (a) comprises conducting the reaction in a multistage system under an oxygen partial pressure gradually decreasing from the first stage to the final stage and at a conversion gradually decreasing from the first stage to the final stage, while controlling the conversion so that the molar ratio of benzenephenone-imines to the copper halide in the final stage is maintained at 1 or more.

According to this embodiment, since the first stage having a higher ratio of the benzophenone-imine to

the copper halide catalyst has a higher permissible water partial pressure of the gaseous phase, it is possible to conduct the reaction under pressure, or to conduct the reaction at a high reaction rate by raising the oxygen concentration in the introduced gas. Further, the reaction can be effected using a small-sized reactor. Furthermore, since the permissible water concentration in the reaction mixture is high, the amount of the gas to be introduced for removal of water needs not to be increased.

On the contrary, in the latter stage in which conversion of the benzophenone-imine has proceeded to a certain extent, the molar ratio of the benzophenone-imine to the copper halide and the permissible water partial pressure become smaller. Therefore, the reaction is effected at a rate lower than that of the former stage by reducing the reaction pressure or the oxygen concentration of the introduced gas. The water partial pressure can easily be reduced below the permissible level with a small amount of gas introduced. The reaction rate does not depend on the concentration of benzophenone-imine and becomes lower towards the latter stages by reducing the oxygen partial pressure. The reduction of the reaction rate in the final stage does not give rise to a serious barrier if a high conversion of benzophenone-imine is provided in the first stage wherein the catalyst has high solubility.

In this embodiment, the reaction is carried out in a multistage system wherein the conversion of imines (on the basis of benzophenone-imines introduced into the first stage) is higher in the first stage than in the final stage, preferably in such a manner that the conversion in the final stage is from 5 to 30%.

Further, since the copper halide is liabley sediment if the molar ratio of the benzophenone-imines to the copper halide is less than 1, conversion of the benzophenone-imine should be stopped before the benzophenone-imine/copper halide molar ratio falls to less than 1. It is preferable that the benzophenone-imine/copper halide molar ratio be adjusted to 1 to 5, and preferably 1 to 3, in the final stage and 2 to 20, and preferably 3 to 10, in the stage or stages before the final stage.

The above-described embodiment of step (a) overcomes various disadvantages resulting from insolubilization and sedimentation of the catalyst, thus making it possible not only to achieve stable continuous reaction at a high conversion of benzophenone-imines but also to reduce the requisite amount of the gas to be introduced. In addition, this embodiment eliminates the necessity to use a large-sized apparatus. Thus, by the above-described embodiment, benzophenone-azines can be prepared with an economic advantage.

The term "multistage reaction system" as used herein means a reaction system composed of two or more reaction chambers through which the oxidation reaction mixture successively passes and each of which maintains a different oxygen pressure. Such a reaction system may comprise a plurality of reaction vessels connected in a series, or a plurality of reaction chambers united in one column. The number of stages is not particularly restricted as long as it is 2 or more, but too many stages make operation complicated. Usually, 2 or 3 stages, and preferably 2 stages, are employed.

In this embodiment, large amounts of the copper halide catalyst bring about high reaction rates but in order to control the molar ratio of benzophenone-imines to copper halide within the above-recited range of from 1 to 5 in the final stage, the finally achieved conversion of benzophenone-imines has to be lowered. In turn, small amounts of the catalyst reduce the reaction rate. Accordingly, the amount of the copper halide catalyst to be used should be determined taking into account the factors affecting the reaction rate, such as temperature, oxygen partial pressure, reaction time, etc., and is usually selected from the above-recited range. The molecular oxygen may be pure oxygen or a mixed gas comprising oxygen and an inert gas, e.g., nitrogen, namely air. The partial oxygen pressure is lower in the final stage than in the first stage within such a range that the oxygen partial pressure in the final stage ranges from 0.1 to 1 atm. and that in the former stage or stages ranges from 0.3 to 10 atm. Oxygen or an oxygen-containing mixed gas is continuously introduced into the reaction system, which is preferably dried.

According to this embodiment, the amount of gas to be introduced should be large enough such that the partial water pressure in each reaction stage should be less than the permissible level in agreement with the benzophenone-imine/copper halide molar ratio of the respective reaction stage. Such an amount varies depending upon the reaction temperature, total pressure, oxygen partial pressure, catalyst concentration, retention time, benzophenone-imine/copper halide molar ratio and the like and, therefore, cannot be definitely determined. However, the total requisite amount of gas to be introduced can greatly be lessened as compared to when the reaction is conducted in one step, because the continuous reaction according to this embodiment can be effected in a multistage system in which different reaction conditions can be applied in conformity with the conversion of benzophenone-imine, i.e., the benzophenone-imines/copper halide molar ratio.

Step (b):

In step (b), the oxidation reaction mixture obtained in step (a) is brought into contact with an aqueous solution containing a hydrohalogenic acid to thereby hydrolyze the benzophenone-azine to produce a hydrazine hydrohalide and, at the same time, to extract and remove the catalyst from the oxidation reaction mixture.

The hydrohalic acid used herein can be hydrochloric acid, hydrobromic acid or hydroiodic acid, with hydrochloric acid being preferred. The concentration of the hydrohalic acid in the aqueous solution usually is from 10 to 60% by weight, and preferably from 20 to 40% by weight. The amount of the hydrohalic acid to be used is usually in the range of from 0.8 to 2 mols per mol of the benzophenone-azines subjected to the

reaction. Too low amounts of the hydrohalic acid result in unfavorably low conversions of benzophenone-azines. On the other hand, too high amounts of the hydrohalic acid cause a large amount of the hydrohalic acid to remain unreacted in the hydrolysis reaction mixture, which leads to by-production of a large amount of salts after the subsequent neutralization.

In cases where the oxidation mixture contains unreacted benzophenone-imines, extraction and removal of the catalyst and hydrolysis of benzophenone-azines are sometimes hindered. Such being the case, it is necessary to use a hydrohalogenic acid in an amount equimolar to the unreacted benzophenone-imines to be supplied in addition to the above-described amount. The temperature, pressure and oil-water contact time employed in step (b) are not particularly restricted, but usually, the temperature is from 60° to 200°C; the pressure is from 0.1 to 10 atm.; and the contact time is from 0.1 to 10 hours.

This step can be carried out either in the presence or absence of air, and either in a batch system or in a continuous system. A counter-current multistage operation may also be employed.

Step (c):

The aqueous solution containing the hydrazine hydrohalide and the catalyst obtained in step (b) is separated from the hydrolysis mixture, and adjusted to a pH of from 3 to 7.

If the separated aqueous solution to be subjected to the subsequent extraction step has a pH value less than 3.0, benzophenones-imine supplied in the subsequent step are conspicuously hydrolyzed to reduce the extraction efficiency.

Therefore, it is necessary to increase the pH value of the aqueous solution up to 3 to 7 with an alkali, e.g., ammonia, sodium hydroxide, potassium hydroxide, calcium hydroxide, sodium carbonate, etc., preferably sodium hydroxide, prior to step (d).

The atmosphere for the pH-adjustment is not particularly restricted, but pH-adjustment is preferably effected in the absence of oxygen in order to avoid decomposition of hydrazines in the aqueous solution as possible. An oxygen-free atmosphere can be achieved by filling the system with inert gas, e.g., nitrogen, helium, hydrogen, methane, ethane, carbon dioxide, etc., or steam, or by sealing the system.

Step (d):

This step comprises contacting the aqueous solution at pH 3 to 7 obtained in step (c) with benzophenone-imines to extract the catalyst.

The copper halide catalyst can easily be extracted upon contact with benzophenone-imines, and the extract is to be separated in the subsequent step (e) and recycled as a catalyst for oxidation of benzophenone-imines.

Conditions for extraction of the catalyst cannot be definitely specified, as they vary depending upon the concentration of the catalyst in the aqueous solution to be extracted, the type of benzophenone-imines, whether or not an organic solvent is used, the type of the aqueous solution, the mode of extraction, and the like. However, extraction can usually be conducted at a temperature of from 0° to 200°C, and preferably from 30° to 150°C, and for a period of from 1 second to 1 hour. The atmosphere employed for the extraction is the same as described in step (c).

The amount of the benzophenone-imines to be used as an extracting solvent in this step may be the whole or a part of the benzophenone-imines which are subjected to the oxidation reaction for obtaining benzophenone-azines. In the latter case, it is also possible that the concentration of the copper halide catalyst present in the benzophenone-imines which have been subjected to extraction is adjusted by addition of the benzophenone-imines which are not subjected to extraction, and the resulting benzophenone-imines solution containing the catalyst is recycled to step (a) for oxidation.

The extraction can be effected in either a batch system or a continuous system. In order to improve the extraction efficiency, counter-current multistage extraction can be appropriately adopted.

Step (e):

In this step, the extracted solution obtained in step (d) is separated into a solution of benzophenone-imines containing the catalyst and an aqueous solution containing the produced hydrazine hydrohalogenide to thereby recover the hydrazine hydrohalogenide as an aqueous solution and, at the same time, the solution of the benzophenone-imines containing the catalyst is recycled to step (a).

The hydrazine hydrohalogenide can be obtained as an aqueous solution by this step. It is also possible for the hydrazine hydrohalide to be converted to hydrazine hydrate by addition of an alkali, followed by distillation to obtain hydrazine hydrate.

The copper halide catalyst thus recovered in an oily layer composed of the benzophenone-imines can be reused as a catalyst for the oxidation of benzophenone-imines with molecular oxygen with no need of any additional treatment.

As described above, the present invention makes it possible to recover and reuse the copper halide catalyst by easy liquid-liquid extraction without requiring any extra treatment. Thus, the present invention provides a highly rationalized process for preparing hydrazine hydrohalides with great industrial advantages.

The present invention will now be illustrated in greater detail with reference to examples wherein percentages are by weight unless otherwise indicated. G designates gauge pressures.

Example 1

(1) Preparation of Benzophenone-azine by Oxidation of Benzophenone-imine

To 300 g of benzophenone-imine solution (benzophenone-imine content: 25.0%; benzophenone content: 75%; imine: 0.414 mol) was added 1.31 g (13.2 mmol) of a cuprous chloride catalyst, and the resulting mixture was stirred at 120°C under atmospheric pressure for 2 hours while blowing oxygen gas thereinto at a rate of 0.5 Nl/min. After the reaction, the reaction mixture was analyzed by gas chromatography and was found to contain benzophenone-imine and benzophenone-azine at concentrations of 1.5% and 23.0%, respectively.

(2) Preparation of Hydrazine Hydrochloride by Hydrolysis of Benzophenone-azine, and Extraction of Catalyst from Oxidation Mixture of Benzophenone-imine

Two hundreds grams of the reaction mixture obtained in (1) above and 30.6 g of a 25% aqueous solution of hydrochloric acid (0.210 mol) were contacted at 100°C for 2 hours under stirring to hydrolyze benzophenone-azine and, at the same time, to extract and remove the catalyst.

The rate of hydrolysis of benzophenone-azine was 92% (decomposed azine: 0.118 mol). After the reaction, the aqueous layer was analyzed and was found to contain 8.7 mmols of cuprous chloride (extractability: 99.0%) and 0.118 mol of hydrazine hydrochloride (yield: 92.0%).

(3) pH-Adjustment of Aqueous Solution Containing Hydrazine Hydrochloride and Catalyst

A 40% aqueous solution of sodium hydroxide was added to the aqueous solution containing hydrazine hydrochloride and the catalyst obtained in (2) above under a nitrogen gas stream to adjust to a pH of 5.

(4) Extraction of Catalyst with Benzophenone-Imine

The aqueous solution having been adjusted to a pH of 5 was contacted with 30 g of benzophenone-imine solution having the same composition as used in (1) above under a nitrogen gas stream at 70°C for 20 seconds, to thereby extract cuprous chloride with the benzophenone-imine solution. This extraction operation was repeated twice.

(5) Separation of Hydrazine Hydrochloride Aqueous Solution and Preparation of Benzophenone-azine Using Catalyst Extract (Benzophenone-imine Solution)

Liquid-liquid separation after the extraction of the step (4) above gave an aqueous solution of hydrazine hydrochloride containing 0.118 mol of hydrazine hydrochloride (yield: 92.0%).

The separated catalyst extract (i.e., a benzophenone-imine solution) was adjusted so as to contain 0.85 g (8.6 mmols) of cuprous chloride in 194 g of benzophenone-imine solution (benzophenone-imine content: 25.0%; benzophenone content: 75%; imine: 0.268 mol) by supplying benzophenone-imine and benzophenone. The thus adjusted solution was stirred at 120°C under atmospheric pressure for 2 hours while blowing oxygen gas at a rate of 0.3 Nl/min. The reaction mixture was analyzed by gas chromatography and was found to contain 1.7% of benzophenone-imine and 23.1% of benzophenone-azine.

Example 2

Benzophenone-azine was produced using the apparatus as shown in Figure.

In each of 2-liter reactors (3) and (9) each equipped with a gas sparger and stirring wings was charged 1,000 g of benzophenone-imine solution (benzophenone-imine: 25.0%; CuCl: 0.4%; the remainder was benzophenone) as a raw material. The reaction was continuously carried out in the two reactors at 120°C and at $49.32 \times 10^4$ Pa (4 Kg/cm$^2$G) in reactor (3) and $1.01 \times 10^5$ Pa (0 Kg/cm$^2$G) in reactor (9).

Benzophenone-imine solution was supplied into reactor (3) through pipe (1) at a rate of 500 g/hr, and air was supplied into reactor (3) through pipe (4) at a rate of 0.85 Nl/min. The reaction mixture was withdrawn from pipe (5). The reaction was continuously carried out so that the raw material resides in reactor (3) for 2 hours in average.

The reaction mixture discharged from reactor (3) was supplied into reactor (9) through pipe (7), and air was supplied therein through pipe (10) at a rate of 0.11 Nl/min. The reaction mixture was discharged through pipe (11). The reaction was continuously carried out so that the raw material resides in reactor (9) for 2 hours. After 48-hour continuous reaction, the reaction mixture discharged from pipe (5) contained 3.8% of benzophenone-imine and 21.2% of benzophenone-azine (yield: 85%) and had a benzophenone-imine/copper molar ratio of 5.0. The reaction mixture discharged from pipe (11) contained 0.9% of benzophenone-imine and 24.2% of benzophenone-azine (yield: 96%) and had a benzophenone-imine/copper molar ratio of 1.2. The water concentrations at pipes for discharging air (2) and (8) were 12 mol% and 13 mol%, respectively.

The resulting reaction mixture was a clear reddish brown solution, and no sedimentation of copper chloride was observed in either of reactors (3) and (9).

Comparative Example 1

The reaction was carried out using only reactor (3) at a pressure of $49.32 \times 10^4$ Pa (4 Kg/cm$^2$G).

The same procedure as described in Example 2 was repeated except that 2,000 g of the starting

benzophenone-imine solution was charged in reactor (3) and retained therein for 4 hours in average while introducing air at a rate of 1.40 Nl/min.

After 48-hour continuous reaction, the reaction mixture withdrawn through pipe (5) contained 2.3% of benzophenone-imine and 22.5% of benzophenone-azine (yield: 90%) and had a benzophenone-imine/copper molar ratio of 3.1. The water concentration in the pipe (2) was 8.0 mol%.

The resulting reaction mixture was opaque and greenish black. Sedimentation of the catalyst was observed in reactor (3), and a part of the sedimented catalyst attached to the wall of reactor (3).

Comparative Example 2

The reaction was continuously conducted using only reactor (3) having a volume of 10 liters at a pressure of 0 $Kg/cm^2G$.

The same procedure as described in Example 2 was repeated except that 5,000 g of the starting benzophenone-imine solution was charged in reactor (3) and retained therein for 10 hours on average while supplying air at a rate of 1.0 Nl/min.

After 48-hour continuous reaction, the reaction mixture from pipe (5) contained 1.2% of benzophenone-imine and 23.8% of benzophenone-azine (yield: 95%) and had an imine/copper molar ratio of 1.6. The water concentration in pipe (2) was 12 mol%. The resulting reaction mixture was a clear reddish brown solution. No sedimentation of the catalyst was observed, but the reaction required a long time and a large-sized reactor.

Comparative Example 3

The reaction was continuously performed using only reactor (3) having a volume of 4 liters at a pressure of 0 $Kg/cm^2G$.

The same procedure as described in Example 2 was repeated except that 2000 g of the starting benzophenone-imine solution was charged in the reactor and retained therein for 4 hours in average while introducing air at a rate of 1.0 Nl/min.

After the reaction was continued for 48 hours, the reaction mixture withdrawn from pipe (5) contained 15.5% of benzophenone-imine and 9.5% of benzophenone-azine (yield: 38%) and had an imine/copper molar ratio of 21.2. The water concentration in pipe (2) was 4.8 mol%. The resulting reaction mixture was a clear reddish brown solution, and no sedimentation of the catalyst was observed.

**Claims**

1. A process for preparing a hydrazine hydrohalide which comprises the steps of:

(a) oxidizing a benzophenone-imine with molecular oxygen in the presence of a copper halide catalyst to prepare a benzophenone-azine;

(b) contacting the oxidation reaction mixture obtained in step (a) with an aqueous solution containing a hydrohalogenic acid at a concentration of from 10 to 60% by weight, thereby hydrolyzing the benzophenone-azine to produce a hydrazine hydrohalide and, at the same time, extracting and removing the catalyst with the hydrohalide-containing aqueous solution from the reaction mixture, to obtain an aqueous solution containing the hydrazine hydrohalide and the catalyst;

(c) separating the aqueous solution containing the hydrazine hydrohalide and catalyst which is obtained in step (b) from the hydrolysis mixture and adjusting the solution to a pH of from 3 to 7 with an alkali;

(d) contacting the aqueous solution having a pH of 3 to 7 obtained in step (c) with benzophenone-imine to extract the catalyst with the benzophenone-imine from said aqueous solution;

(e) separating the catalyst-containing benzophenone-imine solution obtained in step (d) from the aqueous solution containing the hydrazine hydrohalogenide, thereby to recover the hydrazine hydrohalide as an aqueous solution and to recycle the catalyst-containing benzophenone-imine solution to step (a).

2. A process as claimed in Claim 1, wherein step (a) is continuously carried out in a multistage system under an oxygen partial pressure gradually decreasing from the first stage to the final stage and at a conversion gradually decreasing from the first stage to the final stage, while controlling the conversion so that the molar ratio of benzophenone-imine to the copper halide in the final stage is maintained at 1 or more.

3. A process as claimed in Claim 1 or 2, wherein the copper halide in step (a) is cuprous chloride.

4. A process as claimed in Claim 1, 2 or 3, wherein the benzophenone-imine in step (a), (d) or (e) is benzophenone-imine, and the benzophenone-azine in step (a) or (b) is benzophenone-azine.

5. A process as claimed in any preceding claim, wherein step (a) is carried out in the presence of benzophenone as a solvent.

6. A process as claimed in any preceding claim, wherein the aqueous solution in step (b) contains a hydrohalic acid at a concentration of from 20 to 40% by weight.

7. A process as claimed in any preceding claim, wherein the hydrohalic acid in step (b) is used in an amount of from 0.8 to 2 mols per mole of the benzophenone-azine.

8. A process as claimed in any preceding claim, wherein the oxidation reaction mixture obtained in step (a) contains the unreacted benzophenone-imine and the hydrohalic acid in step (b) is used in an

equimolar amount with the unreacted benzophenone-imine and in an amount from 0.8 to 2 mols per mole of the benzophenone-azine.

9. A process as claimed in any preceding claim, wherein the hydrohalic acid in step (b) is hydrochloric acid.

10. A process as claimed in any preceding claim, wherein the alkali in step (c) is ammonia, sodium hydroxide, potassium hydroxide, calcium hydroxide or sodium carbonate.

**Patentansprüche**

1. Verfahren zur Herstellung eines Hydrazinhydrohalogenids, umfassend die Stufen:

(a) Oxidieren eines Benzophenon-Imins mit molekularem Sauerstoff in Gegenwart eines Kupferhalogenidkatalysators unter Herstellung eines Benzophenon-Azins;

(b) Kontaktieren des in Stufe (a) erhaltenen Oxidationsreaktionsgemisches mit einer wäßrigen Lösung, enthaltend eine Halogenwasserstoffsäure mit einer Konzentration von 10 bis 60 Gew.-%, wodurch das Benzophenon-Azin unter Ausbildung von Hydrazinhydrohalogenid hydrolisiert wird und gleichzeitig Extrahieren und Entfernen des Katalysators mit einer einen Halogenwasserstoff enthaltenden wäßrigen Lösung aus dem Reaktionsgemisch unter Erhalt einer wäßrigen Lösung, enthaltend das Hydrazinhydrohalogenid und den Katalysator;

(c) Abtrennung der das Hydrazinhydrohalogenid und den Katalysator enthaltenden in Stufe (b) erhaltenen wäßrigen Lösung aus dem Hydrolysegemisch und Einstellen eines pH-Wertes in der Lösung von 3 bis 7 mit Alkali;

(d) Kontaktieren der in Stufe (c) erhaltenen wäßrigen Lösung mit einem pH-Wert von 3 bis 7 mit Benzophenon-Imin unter Extrahieren des Katalysators mit dem Benzophenon-Imin aus der wäßrigen Lösung;

(e) Abtrennen der in Stufe (d) erhaltenen Katalysator enthaltenden Benzophenon-Imin-Lösung von der wäßrigen, das Hydrazinhydrohalogenid enthaltenden Lösung und Gewinnung des Hydrazinhydrohalogenids als wäßrige Lösung und Rückführen der Katalysator enthaltenden Benzophenon-Imin-Lösung in Stufe (a).

2. Verfahren gemäß Anspruch 1, bei dem die Stufe (a) kontinuierlich in einem Mehrstufensystem unter einem allmählich von der ersten Stufe zu der letzten Stufe abnehmenden Sauerstoffpartialdruck durchgeführt wird, und die Umwandlung allmählich von der ersten Stufe zu der letzten Stufe abnimmt, während man die Umwandlung so einstellt, daß das Mol-Verhältnis des Benzophenon-Imins zu dem Kupferhalogenid in der letzten Stufe bei 1 oder mehr gehalten wird.

3. Verfahren gemäß Anspruch 1 oder 2, bei dem das Kupferhalogenid in Stufe (a) Kupfer (3) chlorid ist.

4. Verfahren gemäß Anspruch 1, 2 oder 3, bei dem das Benzophenon-Imin in Stufe (a), (d) oder (e) Benzophenon-Imin ist, und das Benzophenon-Azin in Stufe (a) oder (b) Benzophenon-Azin ist.

5. Verfahren gemäß einem der vorhergehenden Ansprüche, bei dem die Stufe (a) in Gegenwart von Benzophenon als Lösungsmittel durchgeführt wird.

6. Verfahren gemäß einem der vorhergehenden Ansprüche, bei dem die wäßrige Lösung in Stufe (b) eine Halogenwasserstoffsäure in einer Konzentration von 20 bis 40% enthält.

7. Verfahren gemäß einem der vorhergehenden Ansprüche, bei dem die Halogenwasserstoffsäure in Stufe (b) in einer Menge von 0,8 bis 2 Mol pro Mol des Benzophenon-Azins verwendet wird.

8. Verfahren gemäß einem der vorhergehenden Ansprüche, bei dem das in Stufe (a) erhaltene Oxidationsreaktionsgemisch das nicht-umgesetzte Benzophenon-Imin enthält und die Halogenwasserstoffsäure in Stufe (b) in einer äquimolaren Menge mit dem nicht-umgesetzten Benzophenon-Imin und in einer Menge von 0,8 bis 2 Mol pro Mol des Benzophenon-Azins verwendet wird.

9. Verfahren gemäß einem der vorhergehenden Ansprüche, bei dem die Halogenwasserstoffsäure in Stufe (b) Chlorwasserstoffsäure ist.

10. Verfahren gemäß einem der vorhergehenden Ansprüche, bei dem das Alkali in Stufe (c) Ammoniak, Natriumhydroxid, Kaliumhydroxid, Calciumhydroxid oder Natriumkarbonat ist.

**Revendications**

1. Procédé pour préparer un halogènhydrate d'hydrazine, comprenant les étapes consistant à:

(a) oxyder une benzophénone-imine par de l'oxygène moléculaire, en présence d'un catalyseur à base d'halogénure de cuivre, pour préparer une benzophénone-azine;

(b) mettre le mélange de la réaction d'oxydation, obtenu à l'étape (a), en contact avec une solution aqueuse contenant un hydracide halogéné présent en une concentration de 10 à 60% en poids, de façon à hydrolyser la benzophénone-azine et de produire un halogènhydrate d'hydrazine et, en même temps, de façon à extraire et enlever du mélange réactionnel la solution aqueuse contenant le catalyseur avec l'halogènhydrate, afin d'obtenir une solution aqueuse contenant l'halogènhydrate d'hydrazine et le catalyseur;

(c) séparer la solution aqueuse, contenant l'halogènhydrate d'hydrazine et le catalyseur, que l'on obtient à l'étape (b), du mélange d'hydrolyse et ajuster, le pH de la solution, à l'aide d'une substance alcaline, à une valeur comprise entre 3 et 7;

(d) mettre la solution aqueuse, ayant un pH de 3 à 7, que l'on obtient à l'étape (c), en contact avec de la benzophénone-imine pour extraire le catalyseur avec la benzophénone-imine de ladite solution aqueuse;

(e) séparer la solution de benzophénone-imine, contenant le catalyseur, obtenue à l'étape (d), de la solution aqueuse contenant l'halogènhydrate d'hydrazine, afin de récupérer l'halogènhydrate d'hydrazine, sous forme d'une solution aqueuse, et à recycler la solution de benzophénone-imine, contenant le catalyseur, vers l'étape (a).

2. Procédé tel que revendiqué à la revendication 1, dans lequel on effectue en continu l'étape (a), dans un système à plusieurs étages en opérant sous une pression partielle d'oxygène diminuant progressivement du premier étage vers l'étage final, et à un taux de conversion diminuant progressivement du premier étage vers l'étage final, tout en réglant la conversion de manière à maintenir à une valeur égale ou supérieure à 1 le rapport de la benzophénone-imine à l'halogénure de cuivre dans l'étage final.

3. Procédé tel que revendiqué à la revendication 1 ou 2, dans lequel l'halogénure de cuivre est, dans l'étape (a), du chlorure cuivreux.

4. Procédé tel que revendiqué à la revendication 1, 2 ou 3, dans lequel la benzophénone-imine est, dans l'étape (a), (d) ou (e), de la benzophénone-imine, et la benzophénone-azine est, dans l'étape (a) ou (b), de la benzophénone-azine.

5. Procédé tel que revendiqué dans l'une quelconque des revendications précédentes, dans lequel on effectue l'étape (a) en opérant en présence de benzophénone comme solvant.

6. Procédé tel que revendiqué dans l'une quelconque des revendications précédentes, dans lequel la solution aqueuse contient, dans l'étape (b) un hydracide halogéné présent en une concentration de 20 à 40% en poids.

7. Procédé tel que revendiqué dans l'une quelconque des revendications précédentes, dans lequel on utilise dans l'étape (b) l'hydracide halogéné en une quantité de 0,8 à 2 moles par mole de la benzophénone-azine.

8. Procédé tel que revendiqué dans l'une quelconque des revendications précédentes, dans lequel le mélange de la réaction d'oxydation que l'on obtient à l'étape (a) contient la benzophénone-imine n'ayant pas réagi, et l'on utilise dans l'étape (b) l'hydracide halogéné en une quantité équimolaire par rapport à la benzophénone-imine n'ayant pas réagi et en une quantité de 0,8 à 2 moles per mole de la benzophénone-azine.

9. Procédé tel que revendiqué dans l'une quelconque des revendications précédentes, dans lequel l'hydracide halogéné que l'on utilise dans l'étape (b) est l'acide chlorhydrique.

10. Procédé tel que revendiqué dans l'une quelconque des revendications précédentes, dans lequel la substance alcaline utilisée à l'étape (c) est de l'ammoniaque, de l'hydroxyde de sodium, de l'hydroxyde de potassium, de l'hydroxyde de calcium ou du carbonate de sodium.

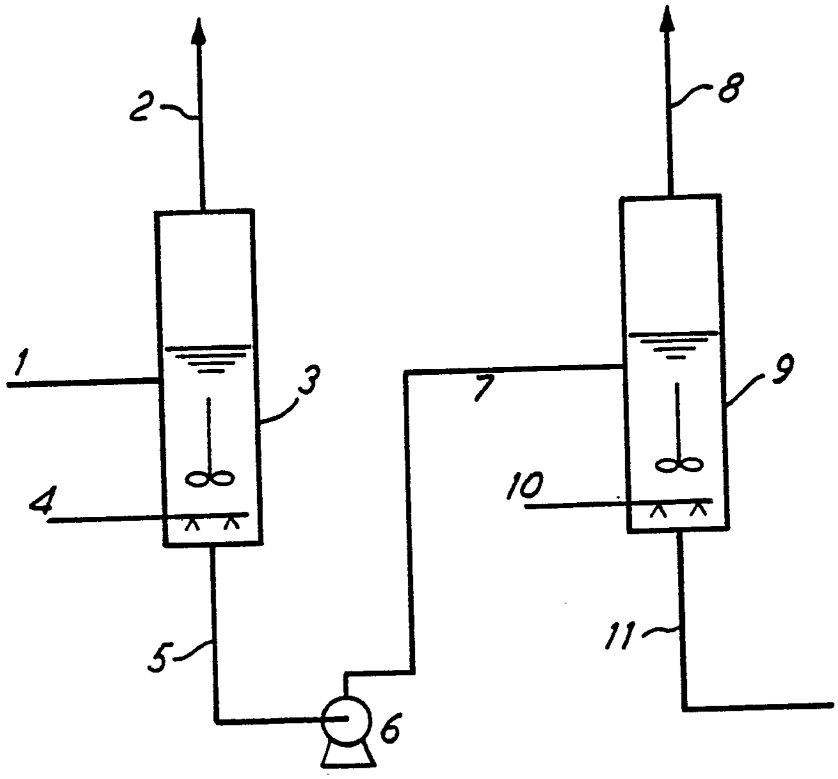
1
2
3
4
5
6
7
8
9
10
11